Europäisches Patentamt

European Patent Office

Office européen des brevets

(11) Publication number: **0 290 707 B1**

# EUROPEAN PATENT SPECIFICATION

(45) Date of publication of patent specification: **22.07.92**

(21) Application number: **87850160.0**

(22) Date of filing: **13.05.87**

(51) Int. Cl.5: **C12N 15/31**, C07K 15/04, C12N 1/20, C12P 21/00, G01N 33/68, A61K 39/02

(54) An immunoglobulin A receptor protein (Arp), cloning and expression thereof.

(43) Date of publication of application:
**17.11.88 Bulletin 88/46**

(45) Publication of the grant of the patent:
**22.07.92 Bulletin 92/30**

(84) Designated Contracting States:
**AT BE CH DE FR GB GR IT LI LU NL SE**

(56) References cited:
EP-A- 0 200 909
WO-A-84/02194
WO-A-85/00832

INFECTION AND IMMUNITY, vol. 55, no. 5, May 1987, pages 1151-1155, American Society for Microbiology; P.H. CLEAT et al.: "Cloning and expression in Escherichia coli of the Ibc protein genes of group B Streptococci: binding of human immunoglobulin A to the beta antigen"

Eur. I. Immunol. 1991.21 : 1481-1490

Eur. Immunol. 1990.20 : 2241-2247

(73) Proprietor: **HighTech Receptor AB**
**Skeppsbron 2**
**S-211 20 Malmö(SE)**

(72) Inventor: **Gunnar, Lindahl**
**Magle lilla kyrkogata 6**
**S-223 51 Lund(SE)**

(74) Representative: **Fagerlin, Heléne et al**
**H. ALBIHNS PATENTBYRA AB P.O. Box 3137**
**S-103 62 Stockholm(SE)**

**Description**

This invention relates to a new protein called Arp and subfragments thereof with affinity for immunoglobulin A, a process for cloning and expression of the protein, the corresponding vectors and hosts, a process for preparing the organism, a method for preparing the protein, a reagent kit and a pharamcuetical composition comprising the protein or fragments thereof.

Bacterial surface proteins with affinity for immunoglobulins are important tools in modern immunology. The best known example of such a protein is protein A from Staphylococcus aureus, which is commonly used for the detection and isolation of IgG.

Bacterial proteins with affinity for other Ig classes than IgG would also be of considerable value as immunological tools. With regard to IgA, it is known that certain streptococcal strains bind IgA (Christensen and Oxelius, Acta Path Microbiol Scand, Sect C, 83, 184 (1975)), and isolation of an IgA-binding protein from group B streptococci has even been reported (Russell-Jones et al, J Exp Med 160, 1467 (1984)). However, the extraction method used by these authors - boiling of bacteria in 2% SDS - is not satisfactory for isolation of sufficient amounts of the protein, and the harshness of the procedure is likely to damage the protein. The protein is reported to have a molecular weight of 130 kDa.

Our invention relates to an IgA receptor from group A streptococci with an apparent molecular weight of about 42 kDa on SDS-PAGE and with a N-terminal sequence of:

$$\text{X-X-X-/Thr-Val-Lys-Ala-Glu-Ser-Ser?/-Thr-Val-}$$
$$\text{-Lys-Ala-Glu-Ser-Ser?/-Thr-Ile-Ser-.}$$

The question mark represents a slight uncertainty in the analysis of the amino acid serine in front of it. The invention also concerns such fragments of this protein that binds to IgA. This new receptor, called protein Arp, binds to IgA but not to IgM, IgD or IgE. There is a weak binding of IgG, but this binding of IgG takes place at a separate site on the protein Arp molecule and does not interfere with the binding of IgA. The gene for protein Arp has been cloned into E. coli, where it is expressed. This allows purification of the protein with gentle procedures.

The invention also concerns a recombinant DNA molecule comprising a vector in which a DNA-sequence encoding for a protein expressible in a microorganism has been inserted, characterized in that the DNA-sequence encodes for protein Arp and/or a fragment of this protein with substantially the same binding properties to immunoglobulin A.

The DNA-sequence may derive from group A streptococci, preferably M type 60 and M type 4. Most preferred are streptococcus AW43 and/or AP4.

The invention further concerns a vector encoding for the protein Arp or fragments thereof, which vector is chosen from plasmids, phage DNA or derivates or fragments thereof or combinations of plasmids and phage DNA and yeast plasmids, such as phage lambda, (specifically phage lambda E4 DSM 4102 deposited 6 April 1987 at Deutsche Sammlung von Mikroorganismen (DSM)).

Examples of vectors which can be used for cloning of DNA-sequences encoding for protein Arp and fragments thereof are bacterial plasmids such as plasmids from E coli, phage DNA, e.g. phage lambda fragments or derivates thereof, such as phage lambda EMBL 3, vectors which are combinations of plasmids and phage DNA, yeast plasmids. The choice of the vector is done according to the microorganism used, i.e. the host.

The invention also concerns a host infected, transformed or transfected with a recombinant DNA molecule comprising a vector in which a DNA-sequence encoding for a protein expressible in a microorganism has been inserted. The inserted DNA is characterized in that the DNA-sequence encodes for the protein Arp and/or a fragment of this protein with substantially the same binding properties to immunoglobulin A. Suitable hosts that can be infected, transformed or transfected with the recombinant DNA molecule according to the invention and thereby express protein Arp or fragments thereof are gram positive or negative bacteria such as E.coli, B.subtilis and yeast cells. All sorts of E.coli stains can be used, preferably E.coli LE 392.

The invention also concerns a process for preparing an infected, transformed or transfected host defined above. Specifically, the invention concerns a process for infecting E.coli with phage lambda carrying the gene for protein Arp (preferably E.coli LE 392 transfected with phage lambda E4).

The invention further concerns a process for preparing protein Arp and subfragments thereof with IgA binding activity, characterized in that an infected, transformed or transfected host defined above is cultivated and the protein isolated.

Preferably E.coli is infected with phage lambda containing the protein Arp encoding DNA-sequence and after lysis, the lysate liquid is separated from debris and purified by affinity chromatography with a ligand that has affinity for protein Arp. The ligand is preferably IgA (serum IgA, secretory IgA, IgA$_1$ or IgA$_2$). E.coli can be any strain, preferably LE 392, and it is grown in broth, preferably in LB broth. Preferably protease inhibitors such as iodoacetic acid and benzamidinechloride are added before the liquid lysate is separated.

The DNA-sequence that encodes for protein Arp or IgA-binding fragments thereof can be isolated from one or several streptococcus strains mentioned above. The streptococcus cell wall is preferably made fragile and lyzed with enzymes, after which the DNA is purified by phenol extraction and density gradient centrifugation.

The streptococcus strains are cultivated in a rich medium, preferably in Todd-Hewitt broth (oxoid). The cell wall can be made fragile by adding cysteine, threonine and glycine to the culture. The bacteria are lyzed by the addition of enzymes attacking the peptidoglycan layer (preferably mutanolysin), followed by sodium dodecyl sulphate (SDS). The DNA is purified by phenol extraction and density gradient centrifugation.

The streptococcal DNA is treated with a restriction enzyme to yield fragments that can be ligated to the vector. The vector with inserted streptococcal DNA is then used to infect, transform or transfect a host cell. Production of protein Arp is tested. When bacteriophage lambda is the vector, this can be done by covering plates with plaques with a nitrocellulose membrane, which is then exposed to radioactive IgA or to IgA followed by peroxidase conjugated anti IgA. Positive reacting clones are collected.

Preferably the streptococcal DNA is treated with Sau 3AI to yield fragments in the size range of 10-20 kB. This DNA is ligated with T4 DNA ligase to lambda EMBL 3 arms which is allowed to infect E.coli P2392 which only allows phages with inserts to form plaques. The plates with plaques are covered with nitrocellulose membranes and analyzed as above, where presence of peroxidase is detected by staining with 3-amino-9-ethyl-carbazol.

Phage clones giving a positive signal contain the protein Arp encoding DNA, which can be cut out with restriction enzymes.

Preferably the DNA-sequence containing the protein Arp gene is isolated by treating DNA from phage lambda E4 (DMS 4102) with Sal I, followed by agarose gel electrophoresis and collection of the insert, with size 15,0 kB.

The DNA-sequence that codes for a protein or a peptide with IgA-binding activity could be any fragment of this insert or any similar nucleotide sequence that codes for and expresses such a protein or peptide or fragments thereof.

The protein or subfragments thereof can be used gent for binding, separation and identification of immunoglobulin A. Since IgA is the predominant antibody in mucous secretions, IgA-binding proteins are of considerable potential interest for the analysis of this important line of host defense.

The invention therefore also concerns a reagent kit containing protein Arp or subfragments thereof.

The new protein can also be used for absorption of immunoglobulin A from various biological specimens, such as the blood of patients with autoimmune disease.

Thus the invention also concerns a pharmaceutical composition containing protein Arp or subfragments thereof as active ingredients, possibly together with pharmaceutically acceptable adjuvants and excipients.

The protein according to the invention can be produced in E.coli, using the cloned gene. In our hands, cloning has been achieved by using bacteriophage lambda as the vector, but since our results show that the protein can indeed be produced in E.coli, it should also be possible to use other vectors than lambda and probably also other hosts than E.coli.

The invention is further described with the accompanied drawings of which

Fig 1 shows the binding of polyclonal IgA and IgG to Streptococcus strain AW43 at different bacterial concentrations,

Fig 2 shows a spot-test demonstrating production of IgA-binding protein by lambda clones. A petri plate with two identical vertical rows of phage spots was used. The type of insert carried by the phage is indicated at the left; the insert originates from the IgA-binding strain AW43 or the IgG-binding strain G148. A nitrocellulose filter was layered on the plate, and then divided into two halves. One filter-half was probed with radiolabelled IgA, the other with IgG followed by autoradiography,

Fig 3 shows analysis of purified protein Arp by SDS-polyacrylamide gel electrophoresis. A crude phage lysate containing protein Arp (lanes 1 and 3) is compared to protein Arp from the same lysate, purified on IgA-Sepharose (lanes 2 and 4). Lane 1 and 2 were stained with Coomassie Brilliant Blue; lanes 3 and 4 were transferred to nitrocellulose membranes and blotted with $^{125}$I-IgA.

Fig 4 shows a dot-binding assay on nitrocellulose, showing binding of $^{125}$I-protein Arp to different proteins. The indicated amounts of protein, diluted in PBS, were applied to a nitrocellulose membrane in

200 $\mu$l aliquots. The membrane was air-dried, probed with $^{125}$I-protein Arp and autoradiographed.

Binding of IgA to group A streptococci

Initial experiments were directed at finding a strain of group A streptococci with high capacity to bind IgA. Therefore a great number of streptococci group A were studied. This was done in binding assays with whole bacteria.

The group A streptococcal strains originate from two different collections. One series of 52 strains (the AP series) was originally received from Dr J Rotta in Prague; these strains all belong to different M types. A second series of 52 strains (the AW series) were obtained form Dr G Kronvall in Stockholm. The strains were grown in Todd-Hewitt broth (oxoid).

The binding test was performed as follows: The protein under study was labelled with carrier-free $^{125}$I (Amersham International, England), using the chloramine T method (Greenwood F C, Hunter, W H and Glover, J S, Biochem J 89 114 (1963)). Free unreacted isotope was separated from protein by gel chromatography on 9 ml columns of Sephadex G-25 (PD-10, Pharmacia, Uppsala, Sweden). The specific activity of the radiolabelled immunoglobulins was 1-3 mCi/mg. Duplicate samples containing 2-5 ng (about $10^4$ cpm) of radiolabelled immunoglobulin in 25 $\mu$l PBSAT (0.12 M NaCl, 0.03 M phosphate, 0.02% NaN$_3$, 0.05% Tween 20, pH 7.2) were mixed with $2 \times 10^8$ bacteria suspended in 200 $\mu$l PBSAT. Radiolabelling was performed with carrier-free [125]I.

All assays were carried out in plastic tubes at room temperature.

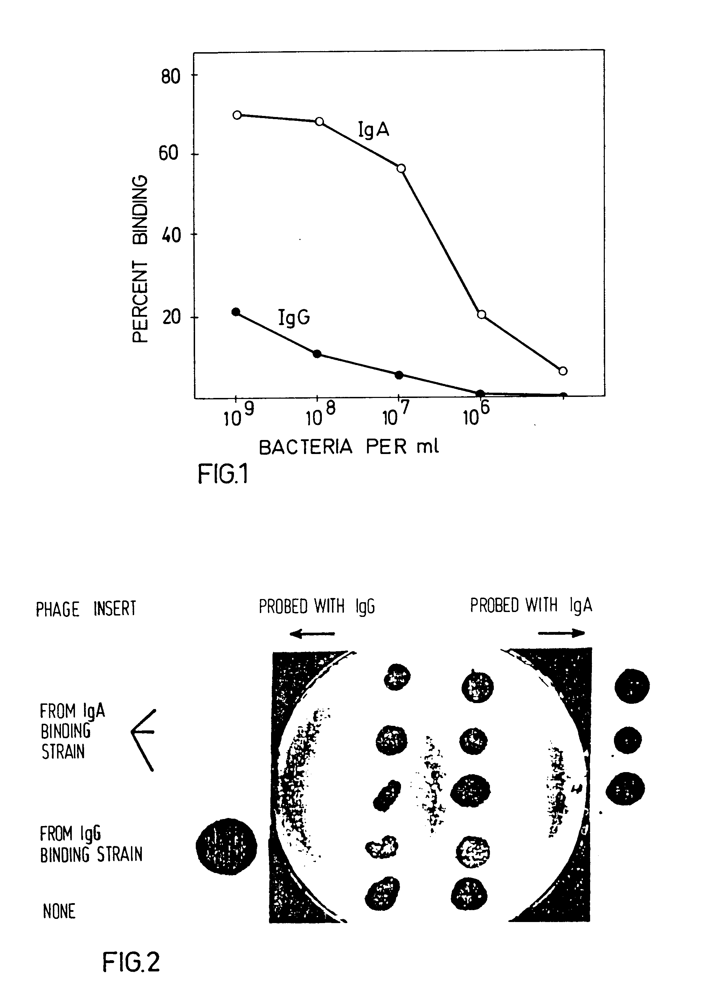

After incubation for 60 min, 2 ml of PBSAT were added to each tube and the bacteria were spun down (2000 x g for 15 min). The supernatant was discharged and the radioactivity in the pellet was measured in a gamma counter. The quantity bound is expressed in per cent of added radioactivity. Non-specific uptake (less than 5%) recorded with the non-binding strain Staphylococcus epidermidis L603 has been deduced. In some experiments, the ability of a bacterial strain to bind labelled protein was tested at different bacterial concentrations (Fig 1). In these experiments the test bacteria were diluted in a suspension of non-binding Staphylococcus epidermidis L603 bacteria, to keep the total number of bacteria in the test mixture constant.

Among 104 strains representing a large number of different M types, 23 strains were found to bind more than 50% of added radiolabelled IgA in the standard binding test described above. The capacity of these strains to bind IgA was then compared in experiments where decreasing amounts of bacteria were mixed with a standard amount of labelled IgA (Fig 1). According to these tests, five of the streptococcal strains had a binding capacity clearly exceeding that of the other strains (data not shown). Two strains were chosen for further study. One of these strains (AW43) belongs to M type 60. The other strain (AP4) belongs to M type 4. The ability of these two strains to bind other immunoglobulins than serum IgA was tested, with very similar results for the two strains (see Table I).

### Table I. Binding of immunoglobulins to two strains of group A streptococci.

| Immunoglobulin | Binding (%) of added immunoglobulin | |
| --- | --- | --- |
| | strain AW43 | strain AP4 |
| IgA, serum | 67 | 60 |
| IgA, secretory | 71 | 62 |
| IgA1 | 62 | 49 |
| IgA2 | 45 | 43 |

| IgG | 21 | 20 |
| IgG1 | 16 | 24 |
| IgG2 | 19 | 23 |
| IgG3 | 11 | 20 |
| IgG4 | 23 | 29 |
| IgM | 2 | 1 |

Serum IgA, secretory IgA, IgG and IgM were polyclonal, the other proteins were monoclonal.

Immunoglobulins were of human origin and polyclonal, unless otherwise stated. Serum IgA, secretory IgA and one monoclonal IgAl protein were from Cooper Biomedical, Malvern, PA. A second monoclonal IgAl protein was from Calbiochem-Behring, San Diego, CA. A third monoclonal IgA protein was purified from the serum of a patient with multiple myeloma, using zone electrophoresis in 0.6% agarose (Johansson, 1972), followed by gel filtration. A monoclonal IgA2 protein was the gift of Dr Anders Grubb, Malmö General Hospital; minor amounts of contaminating IgG and albumin were removed by passage through a small column of protein G-Sepharose, which binds both IgG and albumin. IgG was from AB Kabi, Stockholm, Sweden. Monoclonal IgG of all four subclasses was the gift of Dr Lars Björck. IgM was from Calbiochem-Behring. Monoclonal IgD was the gift of Dr Anders Grubb and monoclonal IgE was the gift of Dr Inge Olsson, University of Lund.

Both strains bind secretory IgA and IgA of the two subclasses. In addition, there is a lower degree of binding of IgG (see also Fig 1). This limited binding of IgG is not due to selective binding of one subclass, since all four subclasses bind equally poorly. There was no significant binding of IgM. The binding of IgA to strain AW43 was further characterized through inhibition tests. Binding of radiolabelled serum IgA could be completely inhibited by the addition of an excess of unlabelled serum IgA or a monoclonal IgAl protein, but unlabelled IgG did not at all inhibit binding. This result suggests that IgA and IgG bind to separate structures on the surface of this bacterial strain.

The following example is given as an illustration only and is not intended to limitate the invention in any way.

## Cloning and expression of the protein Arp gene in E.coli

Strain AW43 was chosen for attempts to purify the IgA-binding structure. Preliminary experiments showed that the binding of IgA could be abolished by pretreatment of the bacteria with any of the proteolytic enzymes pepsin, trypsin or papain, but attempts to demonstrate IgA-binding activity in the solubilized material were unsuccessful. However, these experiments clearly indicated that the binding structure is a protein, which will be called protein Arp.

## Isolation of DNA from Streptococcus AW43

Streptococcal DNA was isolated essentially as described by Spanier J G and Cleary P P, Virology, 130, 514-522 (1983). The method involves preparatory steps in order to make the streptococcal cell wall fragile, which is a prerequisite for efficient lysis. A culture (225 ml) of strain AW43 in Todd-Hewitt broth (Oxoid) (Infusion from 450 g fat-free minced beef grams per litre 10.0,Tryptone 20.0, Dextrose 2.0, Sodium bicarbonate 2.0, Sodium chloride 2.0, Disodium phosphate anhyd. 0.4, pH $7.8 \pm 0.2$) was grown at 37°C by shaking to $2 \times 10^8$/ml, when 13 ml of 10% cysteine and 11 ml of 0.4 M DL-threonine were added. Incubation was continued for 1 h, followed by the addition of 125 ml 15% glycine. After 45 min at 37°C the bacteria were centrifuged down, washed three times with 0.2 M NaAc, and resuspended in 10 ml 0.1 M Tris buffer, pH 8.0, containing 25% glucose and 10 mM EDTA. Mutanolysin (Sigma) was added to a concentration of 50 units/ml, followed by incubation at 37°C for 1 h, when 1.0 ml of 10% SDS (Sodium dodecyl sulphate) was added, causing complete lysis immediately. The lysate was digested with proteinase K (0.1 mg/ml) for 2 h at 37°C, extracted three times with buffer-saturated phenol, and dialyzed against TE buffer (0.01 M Tris HCl,1 mM EDTA; pH 7.4). The DNA was purified further by density gradient centrifugation: 1 g of CsCl and 60 $\mu$l of ethidium bromide (10 mg/ml) were added per ml of DNA in TE, followed by centrifugation at 45,000 rpm for 24 h at 20°C in a Beckman VTi 50 rotor. The band containing streptococcal DNA was visualized with ultraviolet light and recovered with a syringe after puncturing of the wall of the tube. The ethidium bromide was removed by extraction with n-butanol saturated with TE, followed by dialysis of the DNA against TE. The yield of DNA was 0.5 mg.

Cloning of the protein Arp gene in lambda EMBL3

Streptococcal DNA was cloned in the lambda vector EMBL3 (Genofit SA, Geneva) according to Frischauf, A-M, Lehrach, H, Poustka, A, and Murray, N, J Mol Biol, 170, 827-842 (1983). Fifty $\mu$g of DNA from strain AW43 in a volume of 100 $\mu$l was partially digested with 5 units of Sau 3AI for 1 h at 37°C. Agarose gel electrophoresis showed that a large fraction of this DNA was in the desired size range (10 to 20 kB). The cleaved DNA was ligated to lambda EMBL3 arms and packaged in vitro with Gigapack (Genofit SA, Geneva), as recommended by the suppliers.

Restriction enzymes and T4 DNA ligase were purchased from New England Biolabs or from Boehringer Mannheim, and were used as recommended by the suppliers.

About $10^5$ p.f.u. were obtained from ligation of 0.25 $\mu$g of streptococcal DNA. The library was used without amplification and was plated on the P2 lysogenic host strain E.coli P2392 (Genofit SA, Geneva) which only allows lambda phage particles with an insert to form plaques. Plaques were tested for presence of IgA-binding protein with a peroxidase technique: Plates with about 1000 plaques were covered with nitrocellulose filters (Schleicher and Schuell, BA 85) and left at 4°C for 1 h. The filters were then removed and incubated in VBS (10 mM veronal buffer, 0.15 M NaCl, pH 7.4) supplemented with 0.25% gelatin and 0.1% Tween 20, followed by washings for 2x5 min in VBS containing 0.5% Tween 20 ("VBST"). The filters were then incubated for 30 min in VBST containing 10 $\mu$l/ml of IgA, followed by washings 2x5 min in VBST. In the following step the filters were incubated for 30 min in VBST containing peroxidase-conjugated rabbit antihuman IgA (Dakopatts, Denmark; 5 $\mu$l per ml of VBST), followed by washings in VBST, 3x5 min. The filters were finally stained for 5 min at pH 5.1 in 50 ml 50 mM acetate buffer containing 25 $\mu$l of 30% $H_2O_2$ and 2 ml 1% 3-amino-9-ethyl carbazole in acetone. All incubations were done at room temperature. Plaques containing protein Arp appeared as red spots on the filters. Positive clones were plaque purified and retested for protein Arp production as described above, and plate lysates were prepared from verified clones. The frequency of positive clones in the library was approximately 0.05%. Several positive clones were found and three of these were chosen for further study. The screening method used to detect these positive clones was indirect and it was therefore essential to demonstrate directly that the lambda clones produced an IgA-binding protein. This was done by a spot-test method, as shown in Fig 2. A bacterial lawn with spots of the lambda clones was covered with a nitrocellulose filter, allowing adsorption of liberated proteins to the filter. The filter was then analyzed for ability to bind radiolabelled IgA or IgG. Spots of the three lambda clones with inserts from the IgA-binding strain indeed contained material that bound IgA but not IgG. This experiment also includes a control, lambda EMBL3 carrying the gene for streptococcal protein G. As expected, this clone binds IgG, but ot IgA. The absence of IgA-binding activity in this control spot demonstrates that the IgA-binding observed with the three other clones is not due to an artefact or to an E.coli protein, but must be directed by the insert of streptococcal DNA.

The stability of positive clones was tested before they were used further. Due to the variable plaque size typical of bacteriophage lambda, this cannot be done directly on plates with lambda plaques, since small plaques give poor signals in the filter test and may falsely appear as negative. Phage stocks to be tested for stability were instead plated out and 30 plaques (of variable sizes) were each picked to 0.5 ml of broth. These suspensions were then spot-tested on a plate seeded with indicator bacteria, resulting in 30 spots of confluent plaques which were then filter-tested as described above. A clone was considered to be stable when all 30 spots were positive in the test.

Two of the three clones were stable, but the third clone was unstable and threw off non-binding segregants. The two stable clones were studied further. The insert of streptococcal DNA in these clones is flanked by Sal I sites (Frischauf, A-M, Lehrach, H, Poustka, A and Murray, N, J Mol Biol 170, 827-842, (1983).

To characterize the inserts in these lambda clones, DNA was isolated from plate lysates (Maniatis, T, Fritsch, E F and Sambrook, J, Molecular Cloning: A Laboratory Manual, Cold Spring Harbor Laboratory Press, NY, (1982)), using strain E.coli LE392 (Genofit SA, Geneva) as the host. Each phage preparation was prepared from twenty plates with confluent lysis, which were eluted with SM buffer (Maniatis et al 1982). Whole bacteria and debris were removed by centrifugation at 7 000 rpm for 15 min, and the phage then sedimented down by centrifugation for 3 h at 17 000 rpm in a Sorvall SS-34 rotor. The pellets were resuspended in SM and the phage further purified in a CsCl step gradient (Maniatis et al 1982), followed by dialysis against 10 mM NaCl, 50 mM Tris HCl, pH 8.0, 10 mM $MgCl_2$. After addition of EDTA to a final concentration of 20 mM, the preparation was extracted with phenol, dialyzed against TE and concentrated by ethanol precipitation. The yield of DNA was about 100 μg.

DNA from the two clones was then digested with SAl I and analyzed by agarose gel electrophoresis. This demonstrated that the size of one insert was 11.5 kB (λ E2) with one internal Sal I site, whereas the other insert had a size of 15.0 kB (λ E4) with two internal Sal I sites (data not shown). These results confirm that the two clones are of independent origin, which was to be expected from the isolation procedure.

Infection of E.coli with phage λ E4 containing streptococcal DNA

A large phage lysate (1-2 1) was prepared by infection of a logarithmically growing culture of strain E.coli LE392 at 1x10⁸ bacteria/ml in LB broth (Maniatis et al, 1982, grams per litre: Bactotryptone 10, Bacto-yeast extract 5, NaCl 10; pH 7.5) using the λ E4 clone. The multiplicity of infection was about 2x10⁻³. After lysis the protease inhibitors iodoacetic acid and benzamidinechloride were added to a final concentration of 10 mM and the lysate was centrifuged at 4 000 rpm for 1 h at 4°C. The supernatant was used directly for affinity chromatography or frozen and used later; in the latter case the lysate had to be centrifuged again since new visible debris were formed by the freezing and thawing.

Purification of protein Arp by affinity chromatography

Affinity chromatography was performed with IgA coupled to CNBr-activated Sepharose 4B (Pharmacia, Uppsala, Sweden), 1.3 mg IgA per ml packed gel. 7 ml of IgA-Sepharose was added to the centrifuged phage lysate and stirred gently at 4°C for 6 h. The gel was recovered on a sintered glass filter, washed extensively with PBS (about 2 1) and then transferred to a small column. The column (7 ml) was eluted with 0.1 M glycine-HCl, pH 2.0 and fractions (3 ml) were collected. The pH of the fractions was immediately adjusted by the addition of 0.75 ml of 0.5 M phosphate buffer, pH 7.4. Fractions were analyzed for protein concentration by absorbance at 280 nm. All measurable protein was recovered in 2 or 3 fractions, which were pooled, dialyzed against PBS and concentrated by centrifugation in a Centricon 10 microconcentrator (Amicon, Danvers, MA). The IgA-Sepharose was washed with PBS and stored in PBS + 0.05% azide.

Analysis of molecular weight by electrophoresis and western blotting of proteins

Polyacrylamide gel electrophoresis (Bio Rad apparatus) with sodium dodecyl sulphate (SDS-PAGE) was performed in slabs using the buffer system of Laemmli, UK, Nature, 227, 660-685 (1970)). The running gel had a total acrylamide concentration of 10% and the cross-linking was 3.3%. Samples were boiled for 3 min in a solution containing 2% SDS and 5% 2-mercaptoethanol before electrophoresis. A kit from Sigma containing bovine albumin (66 kDa), ovalbumin (45 kDa), glyceraldehyde-3-phosphate (36 kDa), carbonic anhydrase (29 kDa), trypsinogen (24 kDa) and trypsin inhibitor (20 kDa) was used as markers for mol wt. The separated proteins were stained with Coomassie Brilliant Blue R-250 or transferred to nitrocellulose membranes by electrophoretic transfer as described by Towbin, H, Staehlin, T and Gordon, J, Proc Nat Acad Sci USA, 76, 4350-4354 (1979), using a Transblot cell from Bio Rad. To saturate free binding sites, the nitrocellulose membranes were washed 4x20 min at 40°C in 250 ml of 10 mM veronal buffer, 0.15 M NaCl, pH 7,4 (VBS) containing 0.25% gelatin and 0.25% Tween 20. Membranes were then incubated for 3 h at room temperature in 20 ml of VBS containing 0.1% gelatin and radioactive protein (2x10⁵ cpm/ml), followed by washings 4x20 min at room temperature in 250 ml of 1 M NaCl, 10 mM EDTA, 0.25% gelatin and 0.25% Tween 20. After air-drying the membranes were autoradiographed at -70°C using Kodak CEA.C X-ray films and Kodak X-Omat regular intensifying screen.

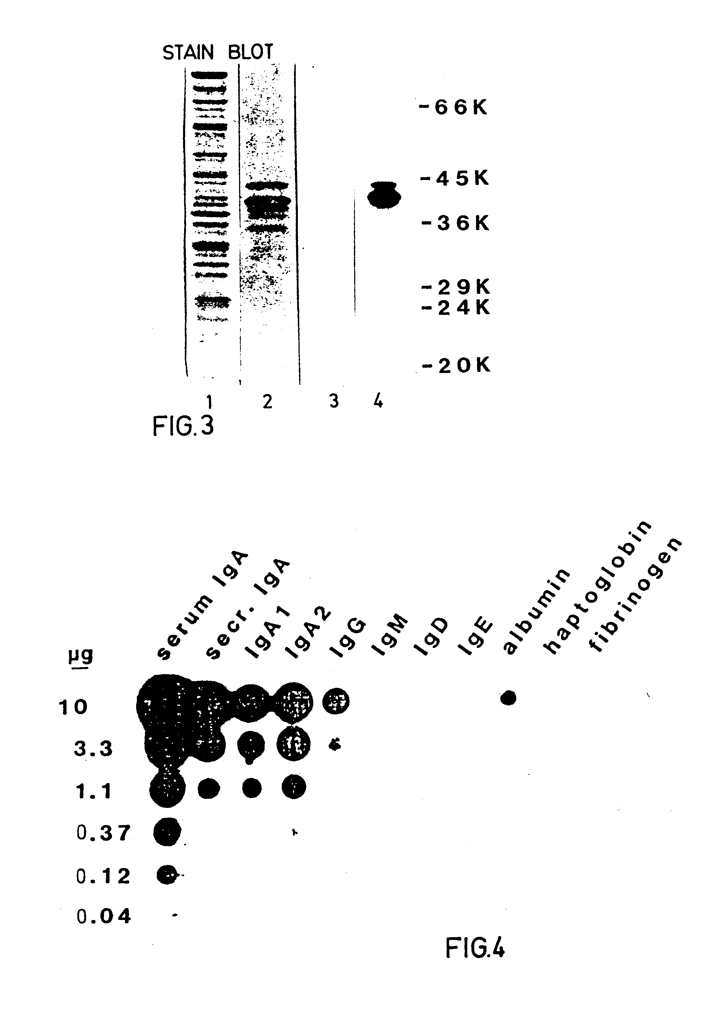

The results are shown in Fig 3. The eluted material was heterogeneous, with a major protein band of 42 kDa, but with additional bands at 45 kDa, 41 kDa and 36 kDa. However, the Western blot showed that each of these bands contained IgA-binding protein. In Fig 3 the IgA-binding is seen clearly only for the major band and one additional band, but prolonged exposure of the film demonstrated IgA-binding also for the other protein bands (but the two bands seen in Fig 3 then merged into one strong band). These results therefore show that the IgA-Sepharose chromatography allowed complete purification of protein Arp in a single step, an about 10 000-fold purification. The yield of protein Arp was about 0.4 mg from 1 1 of phage lysate.

The mol weight pattern seen in Fig 3 was highly reproducible and seen with several different preparations of protein Arp. Furthermore, the same pattern was seen with protein Arp purified from the two independent lambda clones. This strongly indicates that the protein Arp gene was not altered during the cloning procedure.

Detection of IgA bound to nitrocellulose with radiolabelled protein Arp

Radiolabelled protein Arp can be used to detect IgA bound to nitrocellulose in a dot binding assay, as shown in Fig 4, which also demonstrates the specificity of the binding. Serial dilutions of immunoglobulins and some other serum proteins were applied to nitrocellulose membranes using a dot-blot apparatus from Schleicher and Schuell (Dassel, FRG). The membranes were handled as in the blotting procedure described above, except that the final washings were performed with the same buffer as the first washings. The nitrocellulose membrane was incubated with $^{125}$I-protein Arp, followed by autoradiography. This experiment shows that pure protein Arp binds to serum IgA and secretory IgA and to monoclonal IgA proteins of both subclasses. (Binding of two other monoclonal IgAl proteins was demonstrated in other experiments.) There is a much weaker binding to IgG and no binding to IgM, IgD or IgE. A very weak binding was also observed with albumin, but not with haptoglobin or fibrinogen.

**Claims**

**Claims for the following Contracting States : BE, CH/LI, DE, FR, GB, IT, LU, NL, SE**

1. A protein (called Arp) with apparent molecular weight of about 42 kDA (on SDS-PAGE) which specifically binds immunoglobulin A, and subfragments thereof which specifically bind immunoglobulin A, which protein can be produced from phage lambda E4 DSM 4102.

2. Recombinant DNA molecule comprising a vector in which a DNA-sequence encoding for a protein expressible in a host has been inserted, characterized in that the DNA-sequence encodes for the protein according to claim 1 and/or a fragment of this protein with substantially the same binding properties to immunoglobulin A.

3. Recombinant DNA molecule according to claim 2, characterized in that the DNA-sequence derives from A streptococci, preferably Streptococcus AW43 and/or AP4.

4. A vector, characterized in that it encodes for the protein according to claim 1, and preferably is chosen from plasmids, phage-DNA or derivates or fragments thereof or combinations of plasmids and phage-DNA, such as phage lambda, especially phage lambda E4 (DSM 4102).

5. A host, characterized in that it has been infected, transformed or transfected with a recombinant DNA molecule according to claim 2 or 3.

6. A host according to claim 5, characterized in that it is chosen from gram positive or negative bacteria, yeast cells, preferably from E. coli, especially E. coli LE392.

7. A process for preparing an infected, transformed or transfected host according to claims 5 or 6, characterized in that the host is infected, transformed or transfected with a recombinant DNA molecule according to claim 2 or 3.

8. A process for preparing a protein or a fragment thereof according to claim 1, characterized in that an infected, transformed or transfected host is cultivated and the protein isolated.

9. A reagent kit for binding, separation and identification of immunoglobulin A, characterized in that it comprises a protein or a fragment thereof according to claim 1.

10. A pharmaceutical composition, characterized in that it comprises a protein or a fragment thereof according to claim 1.

**Claims for the following Contracting State : AT**

1. A process for preparing a protein (called Arp) with apparent molecular weight of about 42 kDa (on SDS-PAGE) which specifically binds immunoglobulin A, and subfragments thereof which specifically bind immunoglobulin A, which protein can be produced from phage lambda E4 DSM 4102, characterized in cultivating a host infected, transformed or transfected with a recombinant DNA molecule comprising a vector in which a DNA-sequence encoding for the protein or the fragments thereof expressible in the host has been inserted, and isolating the protein.

2. A process according to claim 1, characterized in that the DNA-sequence derives from A streptococci, preferably Streptococcus AW43 and/or AP4.

3. A process according to claim 1, characterized in that the vector is chosen from plasmids, phage-DNA or derivates or fragments thereof or combinations of plasmids and phage-DNA, such as phage lambda, especially phage lambda E4 (DSM 4102).

4. A process according to claim 1, characterized in that the host is chosen from gram positive or negative bacteria, yeast cells, preferably from E. coli, especially E. coli LE392.

5. A process for preparing a pharmaceutical composition, characterized in that a protein or a fragment thereof prepared according to claim 1 is mixed with pharmaceutically acceptable additives.

**Claims for the following Contracting State : GR**

1. A protein (called Arp) with apparent molecular weight of about 42 kDA (on SDS-PAGE) which specifically binds immunoglobulin A, and subfragments thereof which specifically bind immunoglobulin A, which protein can be produced from phage lambda E4 DSM 4102..

2. Recombinant DNA molecule comprising a vector in which a DNA-sequence encoding for a protein expressible in a host has been inserted, characterized in that the DNA-sequence encodes for the protein according to claim 1 and/or a fragment of this protein with substantially the same binding properties to immunoglobulin A.

3. Recombinant DNA molecule according to claim 2, characterized in that the DNA-sequence derives from A streptococci, preferably Streptococcus AW43 and/or AP4.

4. A vector, characterized in that it encodes for the protein according to claim 1, and preferably is chosen from plasmids, phage-DNA or derivates or fragments thereof or combinations of plasmids and phage-DNA, such as phage lambda, especially phage lambda E4 (DSM 4102).

5. A host, characterized in that it has been infected, transformed or transfected with a recombinant DNA molecule according to claim 2 or 3.

6. A host according to claim 5, characterized in that it is chosen from gram positive or negative bacteria, yeast cells, preferably from E. coli, especially E. coli LE392.

7. A process for preparing an infected, transformed or transfected host according to claims 5 or 6, characterized in that the host is infected, transformed or transfected with a recombinant DNA molecule according to claim 2 or 3.

8. A process for preparing a protein or a fragment thereof according to claim 1, characterized in that an infected, transformed or transfected host is cultivated and the protein isolated.

9. A reagent kit for binding, separation and identification of immunoglobulin A, characterized in that it comprises a protein or a fragment thereof according to claim 1.

10. A process for preparing a pharmaceutical composition, characterized in that a protein or a fragment thereof according to claim 1 is mixed with pharmaceutically acceptable additives.

**Revendications**
**Revendications pour les Etats contractants suivants : BE, CH/LI, DE, FR, GB, IT, LU, NL, SE**

1. Protéine (dénommée prA) ayant une masse moléculaire apparente de 42 kDA environ (par électrophorèse sur gel de dodécylsulfate de sodium et de polyacrylamide) et qui fixe spécifiquement l'immunoglobuline A, et ses sous-fragments qui fixent spécifiquement l'immunoglobuline A, cette protéine pouvant être préparée à partir du phage lambda E4 DSM 4102.

2. Molécule d'ADN recombinant, comprenant un vecteur dans lequel a été insérée une séquence d'ADN codant pour une protéine pouvant être exprimée dans un hôte, caractérisée en ce que la séquence d'ADN code pour la protéine de la revendication 1 et/ou pour un fragment de cette protéine, ayant sensiblement les mêmes propriétés de fixation à l'immunoglobuline A.

3. Molécule d'ADN recombinant suivant la revendication 2, caractérisée en ce que la séquence d'ADN dérive de streptocoques A, de préférence de Streptococcus AW43 et/ou AP4.

4. Vecteur, caractérisé en ce qu'il code pour la protéine de la revendication 1, et est choisi de préférence parmi des plasmides, des ADN de phage ou leurs dérivés ou leurs fragments ou des combinaisons de plasmides et d'ADN de phage tels que le phage lambda, en particulier le phage lambda E4 (DSM 4102).

5. Hôte, caractérisé en ce qu'il a été infecté, transformé ou transfecté, par une molécule d'ADN recombinant suivant la revendication 2 ou 3.

6. Hôte suivant la revendication 5, caractérisé en ce qu'il est choisi parmi des bactéries prenant le gram ou ne le prenant pas, des cellules de levure et, de préférence, parmi des E. coli, en particulier E. coli LE392.

7. Procédé de préparation d'un hôte infecté transformé ou transfecté suivant les revendications 5 ou 6, caractérisé en ce qu'il consiste à infecter, à transformer ou à transfecter l'hôte, à l'aide d'une molécule d'ADN recombinant suivant la revendication 2 ou 3.

8. Procédé de préparation d'une protéine ou de l'un de ses fragments suivant la revendication 1, caractérisé en ce qu'il consiste à cultiver un hôte infecté, transformé ou transfecté, et à isoler la protéine.

9. Trousse de réactifs pour la fixation, la séparation et l'identification de l'immunoglobuline A, caractérisée en ce qu'elle comprend une protéine ou l'un de ses fragments suivant la revendication 1.

10. Composition pharmaceutique, caractérisée en ce qu'elle comprend une protéine ou l'un de ses fragments suivant la revendication 1.

**Revendications pour l'Etat contractant suivant : AT**

1. Procédé de préparation d'une protéine (dénommée prA), ayant une masse moléculaire apparente de 42 kDa environ (par électrophorèse sur gel de dodécylsulfate de sodium et de polyacrylamide) et qui fixe spécifiquement l'immunoglobuline A, et de ses sous-fragments qui fixent spécifiquement l'immunoglobuline A, cette protéine pouvant être préparée à partir du phage lambda E4 DSM 4102, caractérisé en ce qu'il consiste à cultiver un hôte infecté, transformé ou transfecté à l'aide d'une molécule d'ADN recombinant comprenant un vecteur dans lequel a été insérée une séquence d'ADN codant pour la protéine ou pour ses fragments, qui peuvent être exprimés dans l'hôte, et à isoler la protéine.

2. Procédé suivant la revendication 1, caractérisé en ce que la séquence d'ADN provient de spectrocoques A, de préférence de spectrococcus AW43 et/ou AP4.

3. Procédé suivant la revendication 1, caractérisé en ce qu'il consiste à choisir le vecteur parmi des plasmides, des ADN de phage ou leurs dérivés ou leurs fragments, ou des combinaisons de plasmides et d'ADN de phage tels que le phage lambda, en particulier le phage lambda E4 (DSM 4102).

4. Procédé suivant la revendication 1, caractérisé en ce qu'il consiste à choisir l'hôte parmi les bactéries prenant le gram ou ne le prenant pas, des cellules de levure, de préférence E. coli, en particulier E. coli LE392.

5. Procédé de préparation d'une composition pharmaceutique, caractérisé en ce qu'il consiste à mélanger une protéine ou l'un de ses fragments préparés suivant la revendication 1, à des additifs acceptables pharmaceutiquement.

**Revendications pour l'Etat contractant suivant : GR**

1. Protéine (dénommée prA) ayant une masse moléculaire apparente de 42 kDA environ (par électrophorèse sur qel de dodécylsulfate de sodium et de polyacrylamide) et qui fixe spécifiquement l'immunoglobuline A, et ses sous-fragments qui fixent spécifiquement l'immunoglobuline A, cette protéine pouvant être préparée à partir du phage lambda E4 DSM 4102.

2. Molécule d'ADN recombinant, comprenant un vecteur dans lequel a été insérée une séquence d'ADN codant pour une protéine pouvant être exprimée dans un hôte, caractérisée en ce que la séquence d'ADN code pour la protéine de la revendication 1 et/ou pour un fragment de cette protéine, ayant sensiblement les mêmes propriétés de fixation à l'immunoglobuline A.

3. Molécule d'ADN recombinant suivant la revendication 2, caractérisée en ce que la séquence d'ADN dérive de streptocoques A, de préférence de Streptococcus AW43 et/ou AP4.

4. Vecteur, caractérisé en ce qu'il code pour la protéine de la revendication 1, et est choisi de préférence parmi des plasmides, des ADN de phage ou leurs dérivés ou leurs fragments ou des combinaisons de plasmides et d'ADN de phage tels que le phage lambda, en particulier le phage lambda E4 (DSM 4102).

5. Hôte, caractérisé en ce qu'il a été infecté, transformé ou transfecté, par une molécule d'ADN recombinant suivant la revendication 2 ou 3.

6. Hôte suivant la revendication 5, caractérisé en ce qu'il est choisi parmi des bactéries prenant le gram ou ne le prenant pas, des cellules de levure et, de préférence, parmi des E. coli, en particulier E. coli LE392.

7. Procédé de préparation d'un hôte infecté transformé ou transfecté suivant les revendications 5 ou 6, caractérisé en ce qu'il consiste à infecter, à transformer ou à transfecter l'hôte, à l'aide d'une molécule d'ADN recombinant suivant la revendication 2 ou 3.

8. Procédé de préparation d'une protéine ou de l'un de ses fragments suivant la revendication 1, caractérisé en ce qu'il consiste à cultiver un hôte infecté, transformé ou transfecté, et à isoler la protéine.

9. Trousse de réactifs pour la fixation, la séparation et l'identification de l'immunoglobuline A, caractérisée en ce qu'elle comprend une protéine ou l'un de ses fragments suivant la revendication 1.

10. Procédé de préparation d'une composition pharmaceutique, caractérisée en ce qu'il consiste à mélanger une protéine ou l'un de ses fragments suivant la revendication 1 à des additifs acceptables pharmaceutiquement.

**Patentansprüche**
**Patentansprüche für folgende Vertragsstaaten: BE, CH/LI, DE, FR, GB, IT, LU, NL, SE**

1. (Mit Art bezeichnetes) Protein einem scheinbaren Molekulargewicht von etwa 42 kDA (gemäß SDS-Page), welches Immunglobulin A spezifisch bindet, und Subfragmente hievon, welche Immunglobulin A spezifisch binden, welches Protein von dem Phagen Lambda E4 DSM 4102 produziert werden kann.

2. Molekül rekombinanter DNA, welches einen Vektor umfaßt, in welchen eine DNA-Sequenz eingesetzt worden ist, welche für ein in eine Wirtsorganismus exprimierbares Protein kodiert, dadurch gekennzeichnet, daß die DNA-Sequenz für das Protein nach Anspruch 1 und/oder für ein Fragment dieses Proteins mit im wesentlichen den gleichen Bindungseigenschaften gegenüber Immunglobulin A kodiert.

3. Molekül rekombinanter DNA nach Anspruch 2, dadurch gekennzeichnet, daß die DNA-Sequenz aus A-Streptokokken, vorzugsweise aus Streptococcus AW43 und/oder AP4, stammt.

4. Vektor, dadurch gekennzeichnet, daß er für das Protein nach Anspruch 1 kodiert, und vorzugsweise aus Plasmiden, Phagen-DNA oder Derivaten oder Fragmenten hievon, oder aus Kombinationen von Plasmiden und Phagen-DNA, wie z.B. jener des Phagen Lambda, insbesondere des Phagen Lambda E4 (DSM 4102), ausgewählt ist.

5. Wirtsorganismus, dadurch gekennzeichnet, daß er mit einem Molekül rekombinanter DNA nach Anspruch 2 oder 3 infiziert, transformiert oder transfiziert worden ist.

6. Wirtsorganismus nach Anspruch 5, dadurch gekennzeichnet, daß er aus grampositiven oder gramnegativen Bakterien, Hefezellen, und vorzugsweise aus E. coli, insbesondere E. coli LE392, ausgewählt ist.

7. Verfahren zur Herstellung eines infizierten, tranformierten oder transfizierten Wirtsorganismus nach Anspruch 5 oder 6, dadurch gekennzeichnet, daß der Wirtsorganismus mit einem Molekül rekombinanter DNA nach Anspruch 2 oder 3 infiziert, transformiert oder transfiziert wird.

8. Verfahren zur Herstellung eines Proteins oder eines Fragmentes hievon, nach Anspruch 1, dadurch gekennzeichnet, daß ein infizierter, transformierter oder transfizierter Wirtsorganismus kultiviert wird, und daß das Protein isoliert wird.

9. Reagenzien-Kit zum Binden, Abtrennen und Identifizieren von Immunglobulin A, dadurch gekennzeichnet, daß es ein Protein oder Fragment hievon nach Anspruch 1 enthält.

10. Pharmazeutische Zusammensetzung, dadurch gekennzeichnet, daß sie ein Protein oder ein Fragment hievon nach Anspruch 1 enthält.

**Patentansprüche für folgenden Vertragsstaat : AT**

1. Verfahren zur Herstellung eines (mit Arp bezeichneten) Proteins mit einem scheinbaren Molekulargewicht von etwa 42 kDa (gemäß SDS-PAGE), welches Protein Immunglobulin A spezifisch bindet, und von Subfragmenten hievon, welche Immunglobulin A spezifisch binden, welches Protein von dem Phagen Lambda E4 DSM 4102 produziert werden kann, dadurch gekennzeichnet, daß ein Wirtsorganismus kultiviert wird, der mit einem Molekül rekombinanter DNA, welches einen Vektor umfaßt, in welchen eine DNA-Sequenz eingesetzt worden ist, welche für ein in dem Wirtsorganismus exprimierbares Protein oder Fragmente hievon kodiert, infiziert, transformiert oder transfiziert worden ist, und daß das Protein isoliert wird.

2. Verfahren nach Anspruch 1, dadurch gekennzeichnet, daß die DNA-Sequenz aus A-Streptokokken, vorzugsweise aus Streptococcus AW43 und/oder AP4, stammt.

3. Verfahren nach Anspruch 1, dadurch gekennzeichnet, daß der Vektor aus Plasmiden, Phagen-DNA oder Derivaten oder Fragmenten hievon, oder aus Kombinationen von Plasmiden und Phagen-DNA, wie z.B. jener des Phagen Lambda, insbesondere des Phagen Lambda E4 (DSM 4102), ausgewählt ist.

4. Verfahren nach Anspruch 1, dadurch gekennzeichnet, daß der Wirtsorganismus aus grampositiven oder gramnegativen Bakterien, Hefezellen, und vorzugsweise aus E. coli, insbesondere E. coli LE392, ausgewählt ist.

5. Verfahren zur Herstellung einer pharmazeutischen Zusammensetzung, dadurch gekennzeichnet, daß ein Protein oder ein Fragment hievon, welches gemäß Anspruch 1 hergestellt worden ist, mit pharmazeutisch annehmbaren Zusatzstoffen vermischt wird.

**Patentansprüche für folgenden Vertragsstaat : GR**

1. (Mit Arp bezeichnetes) Protein mit einem scheinbaren Molekulargewicht von etwa 42 kDA (gemäß SDS-PAGE), welches Immunglobulin A spezifisch bindet, und Subfragmente hievon, welche Immunglobulin A spezifisch binden, welches Protein von dem Phagen Lambda E4 DSM 4102 produziert werden kann.

**2.** Molekül rekombinanter DNA, welches einen Vektor umfaßt, in welchen eine DNA-Sequenz eingesetzt worden ist, welche für ein in einem Wirtsorganismus exprimierbares Protein kodiert, dadurch gekennzeichnet, daß die DNA-Sequenz für das Protein nach Anspruch 1 und/oder für ein Fragment dieses Proteins mit im wesentlichen den gleichen Bindungseigenschaften gegenüber Immunglobulin A kodiert.

**3.** Molekül rekombinanter DNA nach Anspruch 2, dadurch gekennzeichnet, daß die DNA-Sequenz aus A-Streptokokken, vorzugsweise aus Streptococcus AW43 und/oder AP4, stammt.

**4.** Vektor, dadurch gekennzeichnet, daß er für das Protein nach Anspruch 1 kodiert, und vorzugsweise aus Plasmiden, Phagen-DNA oder Derivaten oder Fragmenten hievon, oder aus Kombinationen von Plasmiden und Phagen-DNA, wie z.B. jener des Phagen Lambda, insbesondere des Phagen Lambda E4 (DSM 4102), ausgewählt ist.

**5.** Wirtsorganismus, dadurch gekennzeichnet, daß er mit einem Molekül rekombinanter DNA nach Anspruch 2 oder 3 infiziert, transformiert oder transfiziert worden ist.

**6.** Wirtsorganismus nach Anspruch 5, dadurch gekennzeichnet, daß er aus grampositiven oder gramnegativen Bakterien, Hefezellen, und vorzugsweise aus E. coli, insbesondere E. coli LE392, ausgewählt ist.

**7.** Verfahren zur Herstellung eines infizierten, transformierten oder transfizierten Wirtsorganismus nach Anspruch 5 oder 6, dadurch gekennzeichnet, daß der Wirtsorganismus mit einem Molekül rekombinanter DNA nach Anspruch 2 oder 3 infiziert, transformiert oder transfiziert wird.

**8.** Verfahren zur Herstellung eines Proteins oder eines Fragmentes hievon, nach Anspruch 1, dadurch gekennzeichnet, daß ein infizierter, transformierter oder transfizierter Wirtsorganismus kultiviert wird, und daß das Protein isoliert wird.

**9.** Reagenzien-Kit zum Binden, Abtrennen und Identifizieren von Immunglobulin A, dadurch gekennzeichnet, daß es ein Protein oder Fragment hievon nach Anspruch 1 enthält.

**10.** Verfahren zur Herstellung einer pharmazeutischen Zusammensetzung, dadurch gekennzeichnet, daß ein Protein oder ein Fragment hievon nach Anspruch 1 mit pharmazeutisch annehmbaren Zusatzstoffen vermischt wird.

FIG.1

FIG.2

14

FIG.3

FIG.4